# EUROPÄISCHE PATENTSCHRIFT

(11) **EP 0 905 121 B1**
(45) Veröffentlichungstag und Bekanntmachung des Hinweises auf die Patenterteilung: **03.07.2002**
(21) Anmeldenummer: 98117330.5
(22) Anmeldetag: 12.09.1998
(51) Int. Cl.: C07C 205/37, C07C 205/38, C07C 205/45, C07C 323/09, C07C 201/12, C07C 319/20, C07C 319/14

(54) **o-Nitro(thio)phenolderivate und deren Herstellung**
o-nitro(thio)phenol compounds and their preparation
Composés o-nitro(thio)phénoliques et leur préparation

(30) Priorität: 24.09.1997 DE 19742135
(43) Veröffentlichungstag der Anmeldung: 31.03.1999
(73) Patentinhaber: Infineon Technologies AG, 81669 München (DE)
(72) Erfinder: Sezi, Recai Dr., 91341 Röttenbach (DE); Weber, Andreas Dr., 92289 Ursensollen (DE); Keitmann, Michael, 91074 Herzogenaurach (DE)
(74) Vertreter: Müller - Hoffmann & Partner

(56) Entgegenhaltungen:
- EP-A- 0 331 144
- WO-A-97/23216
- DE-B- 1 178 440
- FR-A- 2 232 313
- FR-A- 2 337 718
- US-A- 2 385 282
- US-A- 3 350 459
- FREEDMAN J ET AL: "The preparation of 3,4-dihydro-1-benzoxepin-5(2H)-ones" J. HETEROCYCL. CHEM. (JHTCAD,0022152X);89; VOL.26 (6); PP.1547-54, XP002087376 Merrell Dow Res. Inst.;Cincinnati; 45215; OH; USA (US)
- CHEMICAL ABSTRACTS, vol. 072, no. 21, 25. Mai 1970 Columbus, Ohio, US; abstract no. 111397, SEVBO D P ET AL: "Synthesis of 3-hydroxy-2',4-diaminodiphenylsulfide and its oxidation to 2-amino-3-phenothiazone" XP002087377 & ZH. ORG. KHIM. (ZORKAE);70; VOL.6 (2); PP.345-7, Leningrad. Tekhnol. Inst. im. Lensoveta;Leningrad; USSR
- DATABASE CROSSFIRE Beilstein Informationssysteme Gmbh, Frankfurt DE, XP002087378 & MONATSH.CHEM., Bd. 5, 1884, Seite 188
- DATABASE CROSSFIRE Beilstein Informationssysteme Gmbh, Frankfurt DE, XP002087379 & J.MED:CHEM., Bd. 8, 1965, Seiten 446-456,
- DATABASE CROSSFIRE Beilstein Informationssysteme Gmbh, Frankfurt DE, XP002087380 & J.CHEM.SOC.,1931, Seite 2100
- DATABASE CROSSFIRE Beilstein Informationssysteme Gmbh, Frankfurt DE, XP002087381 & J.CHEM.SOC.,1933, Seite 660

## Beschreibung

Die Erfindung betrifft neue o-Nitrophenolderivate und o-Nitrothiophenolderivate, die zusammen kurz auch als o-Nitro(thio)phenolderivate bezeichnet werden, sowie Verfahren zu deren Herstellung.

Zur Herstellung von hochtemperaturstabilen Polymeren, wie Polybenzoxazolen (PBO) bzw. deren Vorstufen, sowie zur Herstellung von Hydroxypolyimiden werden Bis-o-aminophenole benötigt (siehe dazu beispielsweise EP 0 264 678 B1 und EP 0 300 326 B1). Diese Monomeren können durch Reduktion der entsprechenden Bis-o-nitrophenole hergestellt werden (siehe dazu EP 0 317 942 A2, SU 1 205 518 A und "Polymer Preprints" 34(1), 1993, Seiten 425 und 426).

Bis-o-nitrophenole können durch Nitrierung von Bisphenolen hergestellt werden. Hierbei entstehen aber Isomere und auch Polynitroverbindungen, was sehr nachteilig ist. Wenn nämlich die Nitrierung nicht vollständig, d.h. zu 100 %, und vollkommen isomerenfrei erfolgt, d.h. nur in o-Stellung zur Hydroxygruppe darf eine Nitrierung erfolgen, dann entstehen nach der Reduktion teilweise Aminophenole, die in der PBO-Vorstufe keine vollständige Cyclisierung mehr zulassen und die Eigenschaften des Polybenzoxazols erheblich beeinträchtigen.

Es ist auch bereits bekannt, für den genannten Zweck Nitrophenolderivate herzustellen, d.h. Bis(o-benzyloxy-nitrophenyl)-Verbindungen, und zwar durch eine nukleophile Substitution (siehe Polymer Preprints", a.a.O.). Dieses Verfahren erfordert aber hohe Temperaturen, nämlich deutlich höhere Temperaturen als 100°C (Lösungen in Dimethylacetamid/Toluol werden zum Rückfluß erhitzt). Hohe Reaktionstemperaturen fördern jedoch Nebenreaktionen, welche die Ausbeute verringern (sie liegt bei maximal 73 %) und die Reinigung des erwünschten Produktes erschweren. Außerdem sind die auf diese Weise hergestellten Bis-o-aminophenole nicht oxidationsstabil.

J. Freedman und K. T. Stuart (J. Heterocyclic Chem., 26, 1547 (1989)) beschreiben die Herstellung von 3,4-Dihydro-1-benzo-oxepin-5(2H)-onen. Als Zwischenprodukt wird die Synthese von 3-Methoxy-4-nitrophenol beschrieben.

In der WO 97/23216 werden Piperidinanaloge beschrieben, die in der 4-Position substituiert sind. Diese Verbindungen können als NMDA-Rezeptorantagonisten verwendet werden. Als ein Zwischenprodukt für die Herstellung derartiger Wirkstoffe wird die Synthese von 4-Benzyloxy-3-nitrophenol beschrieben.

In der US 2,385,282 wird ein Verfahren zur Herstellung von 2-Nitro-3-methoxyphenol beschrieben.

In der FR 2 232 313 werden substituierte Aminoimidazo-[4,5-f] chinoline und Verfahren zu deren Herstellung be-schrieben. Als Zwischenprodukt für die Synthese der Wirkstoffe wird 4-Methoxy-3-nitrophenol beschrieben.

In der FR 2 337 718 werden veretherte Hydroxy-Benzodi-heterozyklen beschrieben, die als Arzneimittel verwendet werden können. Die Synthese der Wirkstoffe verwendet als Zwischenprodukt 2-Allyloxy-3-nitrophenol.

In der DE 1 178 440 werden Verfahren zur Herstellung von 3-Alkoxy-4-nitrophenolen beschrieben.

In der US 3,350,459 werden Mercapto-Nitrophenole sowie Verfahren zu deren Herstellung beschrieben.

Sevbo, D. P. und Ginzburg, O. F. (Zh. Org. Khim. 1970, 6(2), 345 - 7), beschreiben die Kondensation von 2-O₂NC₆H₄Cl und 3,4-(MeO)O₂NC₆H₃SH zu 3-Methoxy-2,4-dinitrodiphenylsulfid und 3,3'-Dimethoxy-4,4'-dinitrodiphenylsulfid, die anschließend zu den entsprechenden Diaminen reduziert werden.

In der EP 0 331 144 A2 werden substituierte para-Nitrophenole beschrieben, wobei einer der Substituenten eine Alkoxygruppe mit zwei bis vier Kohlenstoffatomen sein kann.

Weitere Beispiele für Nitrophenole werden beschrieben in J. Med. Chem., Bd. 8, 1965, Seiten 446 bis 456; J. Chem. Soc. 1931, Seite 2100, J. Chem. Soc., 1933, Seite 660.

Allgemein besteht ein Mangel an geeigneten Aminophenolen - und entsprechend auch an Nitrophenolen - zur Herstellung von Polymeren, welche die stark gestiegenen Anforderungen der Mikroelektronik erfüllen. Außerdem beeinflußt die Art des eingesetzten Aminophenols in starkem Maße das Eigenschaftsspektrum der damit hergestellten PBO-Vorstufe bzw. des Polybenzoxazols. So werden nicht nur das thermische, elektrische und mechanische Verhalten, sondern auch die Löslichkeit und die Hydrolysestabilität sowie zahlreiche weitere Eigenschaften des Poolymers durch das bei der Herstellung verwendete Aminophenol stark beeinflußt.

Aufgabe der Erfindung ist es, o-Nitrophenolderivate und o-Nitrothiophenolderivate bereitzustellen, welche zur Herstellung von o-Aminophenolen bzw. o-Aminothiophenolen geeignet sind, die als Monomere zur Herstellung von Polymer-Vorstufen dienen.

Dies wird erfindungsgemäß durch o-Nitrophenolderivate und o-Nitrothiophenolderivate folgender Struktur erreicht: 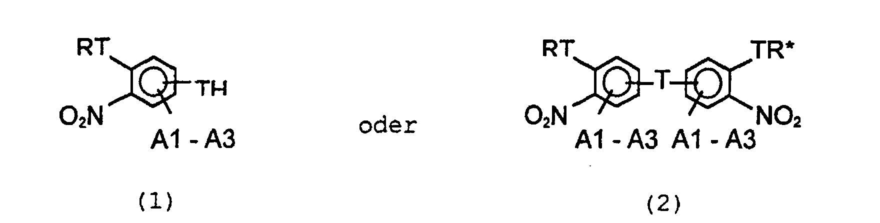 wobei folgendes gilt:
A¹ bis A³ sind - unabhängig voneinander - H, F, CH₃, CF₃, OCH₃, OCF₃, CH₂CH₃, CF₂CF₃, OCH₂CH₃ oder OCF₂CF₃;
T = O oder S;
R = Alkyl, Alkoxyalkyl, Alkenyl, Alkoxyalkenyl, Alkinyl oder Alkoyxyalkinyl mit jeweils maximal sechs C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal vier aliphatischen C-Atomen;
R* = H oder R;
mit der Maßgabe, dass beim o-Nitro(thio)phenolderivat (1) R nicht Alkyl sein darf, wenn A¹ ein F-Atom in m-Stellung zur NO₂-Gruppe ist, A² und A³ die Bedeutung H haben und die TH-Gruppe in o-Stellung zur NO₂-Gruppe ist, und daß weiter, wenn R eine Allyl- oder Benzylgruppe ist, sich beim o-Nitrophenol-derivat (1) die TH-Gruppe in m-Stellung zur TR-Gruppe befindet; und daß weiter beim o-Nitrophenolderivat (1) R nicht Alkyl ist.

Die Charakterisierung "A¹-A³" in den Strukturformeln bedeutet, daß die Nitrophenylgruppen Reste A¹, A² und A³ aufweisen. Dabei können bei (2) die entsprechenden Reste in den beiden Nitrophenylgruppen gleich oder verschieden sein.

Die aus den o-Nitro(thio)phenolderivaten nach der Erfindung herstellbaren o-Amino(thio)phenole führen zu Polymervorstufen, die nach der Cyclisierung Polybenzoxazole bzw. Polybenzothiazole ergeben, welche eine geringe Feuchteaufnahme, eine hohe Temperaturstabilität und einen hohen Planarisierungsgrad aufweisen. Die Polymervorstufen sind in vielen organischen Lösemitteln, wie Aceton, Cyclohexanon, Ethyllactat, Diethylenglykolmono- bzw. -diethylether, N-Methylpyrrolidon und γ-Butyrolacton, sowie in metallionenfreien wäßrig-alkalischen Entwicklern gut löslich. Sie sind deshalb als Basispolymer für positiv photostrukturierbare und wäßrigalkalisch entwickelbare Dielektrika gut geeignet. Die Vorstufen können mittels Schleudertechnik problemlos auf Substrate, wie Siliciumwafer, aufgebracht werden, sie bilden gleichmäßige Filme und lassen sich auf dem Substrat gut cyclisieren.

Die o-Nitro(thio)phenolderivate der Struktur (1) können in der Weise hergestellt werden, daß bei einer Verbindung der Struktur 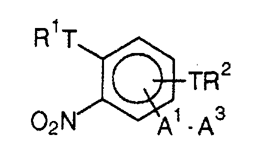 bei einer Temperatur von 20 bis 100°C selektiv der Rest R² abgespalten und durch H ersetzt wird, wobei folgendes gilt:
(a)
   - bei R¹ =: Alkyl, Alkoxyalkyl, Alkenyl, Alkoxyalkenyl, Alkinyl oder Alkoxyalkinyl (mit jeweils maximal 6 C-Atomen)
   - und R² =: Phenyl, Phenacyl oder Benzyl sowie Benzylalkyl, Benzylalkenyl, Benzyloxyalkyl, Benzyloxyalkenyl, Benzylalkoxyalkyl oder Benzylalkoxyalkenyl (mit jeweils maximal vier aliphatischen C-Atomen)
   erfolgt die Spaltung mit Trifluoressigsäure, p-Toluolsulfonsäure, Lithium-, Natrium- oder Kalium-tert.-butoxid oder Lithium-, Natrium- oder Kaliumaluminiumhydrid oder durch Hydrierung mit Wasserstoff an Pd/C;
(b)
   - bei R¹ =: Phenyl, Phenacyl oder Benzyl sowie Benzylalkyl, Benzylalkenyl, Benzyloxyalkyl, Benzyloxyalkenyl, Benzylalkoxyalkyl oder Benzylalkoxyalkenyl (mit jeweils maximal vier aliphatischen C-Atomen)
   - und R² =: Alkyl, Alkoxyalkyl, Alkenyl, Alkoxyalkenyl, Alkinyl oder Alkoxyalkinyl (mit jeweils maximal 6 C-Atomen)
   erfolgt die Spaltung mit einem Metall oder einer Metallverbindung in einem protischen Lösemittel.

Vorzugsweise haben die Reste R¹ und R² folgende Bedeutung: Methyl, Ethyl, Isopropyl, Allyl und Methoxymethyl bzw. Phenyl, Phenacyl (d.h. C₆H₅-CO-CH₂-), Benzyl und Benzyloxymethyl.

Die zur selektiven Spaltung eingesetzten Metalle sind vorzugsweise Ni, Se, Rh, Pd, Pt und Hg, die Metallverbindungen können Salze, metallorganische Verbindungen und Komplexverbindungen, wie (CH₃COO)₂Pd(C₆H₅)₃, sein. Vorteilhaft erfolgt die Spaltung mit Pd/C (Palladium/Kohlenstoff), SeO₂ oder HgCl.

Die Nitroverbindung der vorstehend wiedergegebenen Struktur kann aus der entsprechenden Dihalogenverbindung (X anstelle von R¹T und R²T) durch Umsetzung mit Verbindungen R¹TH und R²TH, beispielsweise Allylalkohol und Benzylalkohol, oder mit entsprechenden Alkalisalzen hergestellt werden.

Alternativ können o-Nitro(thio)phenolderivate der Struktur (1) in der Weise hergestellt werden, daß eine Verbindung der Struktur 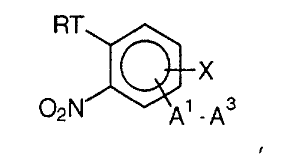 wobei R die angegebene Bedeutung hat und X Halogen ist, mit einem Überschuß eines basischen Agens TH⁻ (mit T = O oder S) bei einer Temperatur von 20 bis 100°C umgesetzt wird.

Das basische Agens kann ein Alkali- oder Erdalkalimetallhydroxid, eine wäßrige Lösung einer tertiären Stickstoffbase, wie Tetramethylammoniumhydroxid, oder eine wäßrige Lösung eines Hydrogensulfids, wie Natriumhydrogensulfid, sein.

Wird das basische Agens nicht im Überschuß, sondern in einer stöchiometrischen Menge eingesetzt, dann entsteht - bei sonst gleichen Bedingungen - ein o-Nitro(thio)phenolderivat der Struktur (2) mit R* = R. Zur Herstellung von Verbindungen, bei denen die beiden Nitrophenylgruppen unterschiedliche Reste A¹ bis A³ aufweisen, werden entsprechend unterschiedliche halogenhaltige Nitroverbindungen eingesetzt.

Die Herstellung von o-Nitro(thio)phenolderivaten der Struktur (2) mit R* = H erfolgt in der Weise, daß ein o-Nitro(thio)phenolderivat der Struktur (1) mit einer Verbindung der Struktur 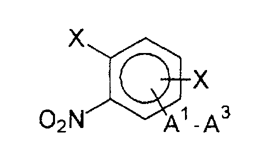 umgesetzt wird, wobei X Halogen ist,
und daß nachfolgend durch Umsetzung mit einem Überschuß eines basischen Agens TH⁻ (mit T = oder S) bei einer Temperatur von 20 bis 100°C das Halogen X in o-Stellung zur NO₂-Gruppe durch TH ersetzt wird.

Die Umsetzungen werden im allgemeinen in einem Lösemittel durchgeführt. Geeignete Lösemittel sind beispielsweise Dimethylformamid, Dimethylacetamid, Dimethylsulfoxid, N-Methylpyrrolidon und γ-Butyrolacton. Die o-Nitro(thio)phenolderivate werden mit einer Ausbeute ≥ 85 % erhalten.

Die Herstellung von Bis-o-amino(thio)phenolen aus den o-Nitro(thio)phenolderivaten nach der Erfindung ist in der gleichzeitig eingereichten deutschen Patentanmeldung Akt.Z. 197 42 196.2 - "Bis-o-amino(thio)phenole und deren Herstellung" (GR 97 P 3684) beschrieben.

Die o-Nitro(thio)phenolderivate nach der Erfindung eignen sich auch zur Herstellung von o-Amino(thio)phenol-carbonsäuren, die ebenfalls zur Herstellung von Polybenzoxazolen und Polybenzothiazolen bzw. den entsprechenden Polymervorstufen dienen. Die Herstellung von o-Amino(thio)phenolcarbonsäuren ist in der gleichzeitig eingereichten deutschen Patentanmeldung Akt.Z. 197 42 194.6 - "o-Amino(thio)phenolcarbonsäuren und deren Herstellung" (GR 97 P 3689) beschrieben.

Anhand von Ausführungsbeispielen soll die Erfindung noch näher erläutert werden.

### Beispiel 1

### Herstellung von 2-Benzyloxy-4-fluor-nitrobenzol (5-Fluor-2-nitrophenyl-benzylether)

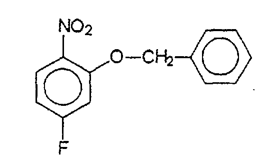

31,4 g 5-Fluor-2-nitrophenol (0,2 mol) und 34,3 g Benzylbromid (0,2 mol) werden in einem 500 ml-Dreihalskolben mit Rückflußkühler, Rührer und Stickstoffzuleitung in 200 ml trockenem Acetonitril gelöst. Nach der Zugabe von 60 g Kaliumcarbonat (0,43 mol) wird die Lösung in einem regelbaren Ölbad 2 h unter Rückfluß erhitzt. Danach läßt man die Reaktionslösung auf Raumtemperatur abkühlen und filtriert den Rückstand über ein Faltenfilter ab. Die erhaltene Lösung wird dann in einem Rotationsverdampfer auf die Hälfte eingeengt und über Nacht bei Raumtemperatur stehengelassen; dabei fällt das gelborangefarbene Reaktionsprodukt kristallin aus. Das Reaktionsprodukt wird dann über ein Faltenfilter abfiltriert, in Ethanol umkristallisiert und anschließend in einem Vakuumschrank 48 h unter Stickstoff bei 40°C/10 mbar getrocknet.

Charakterisierung:
- Massenspektrum: Molekülpeak bei 247
- Elementaranalyse:

| | | | |
|---|---|---|---|
| Theoretischer Wert (in %) | C: 63,2 | H: 4,1 | N: 5,7 |
| Ermittelter Wert (in %) | C: 62,9 | H: 3,9 | N: 5,8 |

- Schmp: 55°C.

### Beispiel 2

### Herstellung von 2-Benzyloxy-4-hydroxy-nitrobenzol

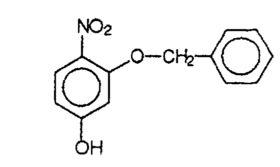

39,5 g des entsprechend Beispiel 1 hergestellten 2-Benzyloxy-4-fluor-nitrobenzol (0,16 mol) werden in einem Dreihalskolben mit Rückflußkühler, Rührer und Stickstoffzuleitung zu einem Gemisch aus einer wäßrigen Kaliumhydroxidlösung, hergestellt aus 27,2 g Kaliumhydroxid (0,48 mol) und 180 ml destilliertem Wasser, und 150 ml Dimethylsulfoxid gegeben, dann wird auf 100°C erhitzt. Nach 4 h läßt man die Reaktionslösung auf Raumtemperatur abkühlen und filtriert den Rückstand über ein Faltenfilter ab. Die dunkelorangefarbene Lösung wird dann zu 800 ml Wasser gegeben und unter starkem Rühren mit konzentrierter Salzsäure bis zur sauren Reaktion versetzt. Innerhalb von ca. 1 h fällt ein weißgelbliches flockiges Reaktionsprodukt aus, das über einen Büchnertrichter abfiltriert und dreimal mit Wasser gewaschen wird. Das Reaktionsprodukt wird in Ethanol umkristallisiert und anschließend in einem Vakuumschrank 48 h unter Stickstoff bei 40°C/10 mbar getrocknet.

Charakterisierung:
- Massenspektrum: Molekülpeak bei 245
- Elementaranalyse:

| | | | |
|---|---|---|---|
| Theoretischer Wert (in %) | C: 63,7 | H: 4,5 | N: 5,7 |
| Ermittelter Wert (in %) | C: 63,4 | H: 4,4 | N: 5,9 |

- Schmp: 117°C.

### Beispiel 3

### Herstellung des Kaliumsalzes von 2-Benzyloxy-4-hydroxy-nitrobenzol

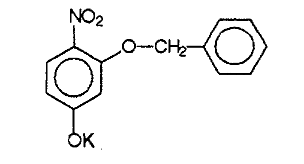

40 g des entsprechend Beispiel 2 hergestellten 2-Benzyloxy-4-hydroxy-nitrobenzol (0,163 mol) werden in einem Dreihalskolben mit Rückflußkühler, Rührer und Stickstoffzuleitung in 300 ml Tetrahydrofuran gelöst. Zur Lösung werden bei Raumtemperatur unter starkem Rühren portionsweise 19,2 g Kaliumtert.-butoxid (0,171 mol) gegeben. Es setzt eine stark exotherme Reaktion ein, in deren Verlauf das Kaliumsalz ausfällt. 15 min nach der Zugabe des Kalium-tert.-butoxid wird zur vollständigen Umsetzung noch 1 h unter Rückfluß erhitzt. Dann läßt man auf Raumtemperatur abkühlen, filtriert das Reaktionsprodukt über einen Büchnertrichter ab und spült dreimal mit einem Gemisch von Tetrahydrofuran und Petroleumbenzin (Volumenverhältnis 1:1) nach. Anschließend wird das Reaktionsprodukt in einem Vakuumschrank 48 h unter Stickstoff bei 40°C/10 mbar getrocknet.

Charakterisierung:
- Massenspektrum: Molekülpeak bei 283
- Elementaranalyse:

| | | | |
|---|---|---|---|
| Theoretischer Wert (in %) | C: 55,1 | H: 3,6 | N: 4,9 |
| Ermittelter Wert (in %) | C: 55,3 | H: 3,7 | N: 4,8 |

### Beispiel 4

### Herstellung von 1-Benzyloxy-4-nitro-2,3,5,6-tetrafluorbenzol

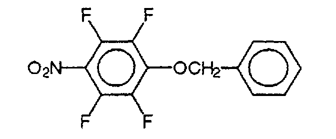

In einem Dreihalskolben mit Rührer werden 21,3 g Pentafluornitrobenzol (0,1 mol) und 12 g Benzylalkohol (0,11 mol, d.h. 10 %iger Überschuß) in 200 ml trockenem γ-Butyrolacton gelöst. Dann gibt man portionsweise 30 g Kaliumcarbonat (0,22 mol) hinzu und rührt das Gemisch 24 h bei Raumtemperatur. Danach wird die Reaktionslösung über ein Faltenfilter abfiltriert und das Rohprodukt mit 200 ml Essigsäureethylester und 300 ml Wasser ausgeschüttelt. Die organische Phase wird dreimal mit Wasser gewaschen, über Natriumsulfat getrocknet und in einem Rotationsverdampfer auf die Hälfte eingeengt. Aus der anfangs zähen Lösung kristallisiert das Reaktionsprodukt aus. Das Reaktionsprodukt wird dann in einem Gemisch von Methylenchlorid und Petroleumbenzin (Volumenverhältnis 1:1) umkristallisiert und anschließend in einem Vakuumschrank 24 h unter Stickstoff bei 40°C/10 mbar getrocknet.

Charakterisierung:
- Massenspektrum: Molekülpeak bei 301
- Elementaranalyse:

| | | | |
|---|---|---|---|
| Theoretischer Wert (in %) | C: 51,8 | H: 2,3 | N: 4,7 |
| Ermittelter Wert (in %) | C: 51,8 | H: 2,3 | N: 4,7 |

### Beispiel 5

### Herstellung von 1-Benzyloxy-3-(prop-2-enyloxy)-4-nitro-2,5,6-trifluorbenzol

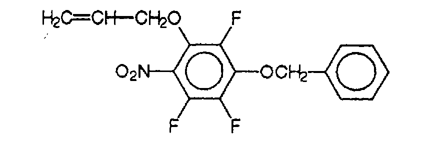

In einem Dreihalskolben mit Rückflußkühler und Rührer werden 30,1 g des entsprechend Beispiel 4 hergestellten 1-Benzyloxy-4-nitro-2,3,5,6-tetrafluorbenzol (0,1 mol) in 250 ml trockenem Acetonitril gelöst und zur Lösung werden portionsweise 8 g Natrium-prop-2-enolat (0,1 mol) gegeben. Man rührt 1 h bei Raumtemperatur und erhitzt dann das Reaktionsgemisch 24 h unter Rückfluß. Danach wird die Reaktionslösung über ein Faltenfilter abfiltriert, und das Rohprodukt wird mit 150 ml Essigsäureethylester und 300 ml Wasser ausgeschüttelt. Die organische Phase wird dreimal mit Wasser gewaschen, über Natriumsulfat getrocknet und in einem Rotationsverdampfer auf die Hälfte eingeengt. Das ausgefallene Reaktionsprodukt wird dann in einem Gemisch von n-Hexan und Methylenchlorid (Volumenverhältnis 1:1) umkristallisiert und anschließend in einem Vakuumschrank 48 h unter Stickstoff bei 40°C/10 mbar getrocknet.

Charakterisierung:
- Massenspektrum: Molekülpeak bei 339
- Elementaranalyse:

| | | | |
|---|---|---|---|
| Theoretischer Wert (in %) | C: 56,6 | H: 3,6 | N: 4,1 |
| Ermittelter Wert (in %) | C: 56,5 | H: 3,5 | N: 4,2 |

### Beispiel 6

### Herstellung von 3-(Prop-2-enyloxy)-4-nitro-2,5,6-trifluorphenol

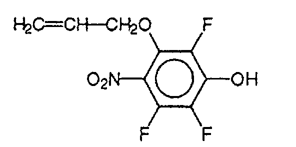

In einem Dreihalskolben mit Rückflußkühler und Rührer werden 33,9 g des entsprechend Beispiel 5 hergestellten 1-Benzyloxy-3-(prop-2-enyloxy)-4-nitro-2,5,6-trifluorbenzol (0,1 mol) in 120 ml Trifluoressigsäure gelöst, dann wird 4 h unter Rückfluß erhitzt. Danach läßt man die Reaktionslösung auf Raumtemperatur abkühlen und schüttelt das Rohprodukt mit 200 ml Essigsäureethylester und 300 ml Wasser aus. Die organische Phase wird dreimal mit Wasser gewaschen, über Natriumsulfat getrocknet und in einem Rotationsverdampfer auf die Hälfte eingeengt. Das ausgefallene Reaktionsprodukt wird dann in einem Gemisch von n-Hexan und Methylenchlorid (Volumenverhältnis 1:1) umkristallisiert und anschließend in einem Vakuumschrank 48 h unter Stickstoff bei 40°C/10 mbar getrocknet.

Charakterisierung:
- Massenspektrum: Molekülpeak bei 249
- Elementaranalyse:

| | | | |
|---|---|---|---|
| Theoretischer Wert (in %) | C: 43,4 | H: 2,4 | N: 5,6 |
| Ermittelter Wert (in %) | C: 43,3 | H: 2,5 | N: 5,6 |

### Beispiel 7

### Herstellung von Bis(3-benzyloxy-4-nitro-phenyl)-ether

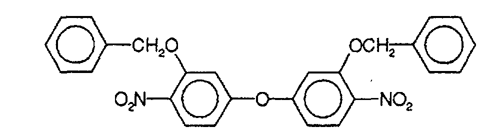

In einem Dreihalskolben mit Rückflußkühler und Rührer werden 400 g Dimethylsulfoxid zusammen mit einer Lösung von 37 g Kaliumhydroxid (0,66 mol) in 400 ml Wasser auf 100°C erwärmt. Dann werden portionsweise 163 g des entsprechend Beispiel 1 hergestellten 5-Fluor-2-nitrophenyl-benzylether (0,66 mol) zugegeben und nachfolgend wird 8 h auf 100°C erwärmt. Nach dem Abkühlen auf Raumtemperatur scheidet sich ein farbloses Reaktionsprodukt ab, das abfiltriert, mit Wasser gewaschen und getrocknet wird. Anschließend wird das Reaktionsprodukt aus Ethylacetat umkristallisiert.

Charakterisierung:
- Massenspektrum: Molekülpeak bei 472
- Schmp: 119°C.

### Beispiel 8

### Herstellung von Bis(4-amino-3-hydroxy-phenyl)-ether

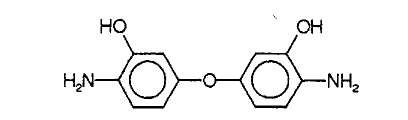

Hierbei werden alle Arbeitsschritte mit entgastem Lösemittel unter Schutzgas durchgeführt.
23,6 g des entsprechend Beispiel 7 hergestellten Bis-(3-benzyloxy-4-nitro-phenyl)-ether (0,05 mol) werden in 200 ml Tetrahydrofuran gelöst, und zu der Lösung werden 2,36 g Pd/C (Palladium/Kohlenstoff) gegeben. Dann wird bei Raumtemperatur in einem Autoklaven mit Wasserstoff bei einem Druck von 2 bar hydriert. Die erhaltene Mischung wird abfiltriert und das Lösemittel zu etwa zwei Drittel abdestilliert; dabei beginnt sich ein farbloses Produkt abzuscheiden. Dieses Produkt wird abgesaugt, mit kaltem Tetrahydrofuran gewaschen und im Vakuum getrocknet. Aus der Mutterlauge läßt sich durch weiteres Einengen eine zweite Kristallfraktion gewinnen.

Charakterisierung:
- Massenspektrum: Molekülpeak bei 232
- Elementaranalyse:

| | | | |
|---|---|---|---|
| Theoretischer Wert (in %) | C: 62,1 | H: 5,2 | N: 12,1 |
| Ermittelter Wert (in %) | C: 61,8 | H: 5,4 | N: 12,1 |

- Schmp: 209°C.

### Beispiel 9

### Herstellung von 1-(4-Nitro-2,3,5,6-tetrafluor-phenoxy)-3-(prop-2-enyloxy)-4-nitro-2,5,6-trifluorbenzol

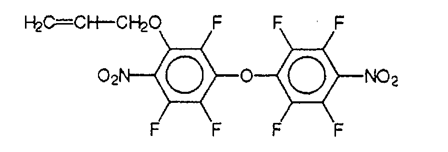

In einem Dreihalskolben mit Rührer werden 24,9 g des entsprechend Beispiel 6 hergestellten 3-(Prop-2-enyloxy)-4-nitro-2,5,6-trifluorphenol (0,1 mol) und 21,3 g Pentafluornitrobenzol (0,1 mol) in 300 ml γ-Butyrolacton gelöst. Dann gibt man portionsweise 30 g Kaliumcarbonat (0,22 mol) hinzu und rührt das Gemisch 24 h bei Raumtemperatur. Danach wird die Reaktionslösung über ein Faltenfilter abfiltriert und das Rohprodukt mit 200 ml Essigsäureethylester und 300 ml Wasser ausgeschüttelt. Die organische Phase wird dreimal mit Wasser gewaschen, über Natriumsulfat getrocknet und in einem Rotationsverdampfer auf die Hälfte eingeengt. Das ausgefallene Rohprodukt wird dann in einem Gemisch von Methylenchlorid und Petroleumbenzin (Volumenverhältnis 1:1) umkristallisiert und anschließend in einem Vakuumschrank 48 h unter Stickstoff bei 40°C/10 mbar getrocknet.

Charakterisierung:
- Massenspektrum: Molekülpeak bei 442
- Elementaranalyse:

| | | | |
|---|---|---|---|
| Theoretischer Wert (in %) | C: 40,7 | H: 1,1 | N: 6,3 |
| Ermittelter Wert (in %) | C: 40,7 | H: 1,0 | N: 6,3 |

### Beispiel 10

### Herstellung von 1-(3-Hydroxy-4-nitro-2,5,6-trifluor-phenoxy)-3-(prop-2-enyloxy)-4-nitro-2,5,6-trifluorbenzol

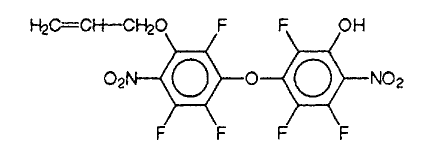

In einem Dreihalskolben mit Rückflußkühler und Rührer werden 44,2 g des entsprechend Beispiel 9 hergestellten 1-(4-Nitro-2,3,5,6-tetrafluor-phenoxy)-3-(prop-2-enyloxy)-4-nitro-2,5,6-trifluorbenzol (0,1 mol) in einem Gemisch von 200 ml Dimethylsulfoxid und 30 ml Wasser gelöst. Zur Lösung werden portionsweise 15 g Kaliumcarbonat (0,11 mol) und 10 g Kaliumhydrogencarbonat (0,1 mol) gegeben, dann wird 24 h bei 80°C gerührt. Danach wird die Reaktionslösung über ein Faltenfilter abfiltriert und das Rohprodukt mit 200 ml Essigsäureethylester und 400 ml Wasser ausgeschüttelt. Die organische Phase wird dreimal mit Wasser gewaschen, über Natriumsulfat getrocknet und in einem Rotationsverdampfer auf die Hälfte eingeengt. Das ausgefallene Reaktionsprodukt wird dann in einem Gemisch von Methylenchlorid und Petroleumbenzin (Volumenverhältnis 1:1) umkristallisiert und anschließend in einem Vakuumschrank 48 h unter Stickstoff bei 40°C/10m bar getrocknet.

Charakterisierung:
- Massenspektrum: Molekülpeak bei 440
- Elementaranalyse:

| | | | |
|---|---|---|---|
| Theoretischer Wert (in %) | C: 40,9 | H: 1,4 | N: 6,4 |
| Ermittelter Wert (in %) | C: 40,9 | H: 1,4 | N: 6,3 |

### Beispiel 11

### Herstellung von Bis(3-hydroxy-4-amino-2,5,6-trifluor-phenyl)-ether

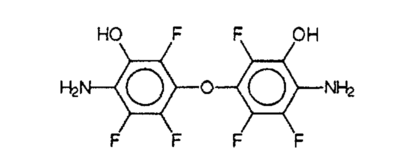

44 g des entsprechend Beispiel 10 hergestellten 1-(3-Hydroxy-4-nitro-2,5,6-trifluor-phenoxy)-3-(prop-2-enyloxy)-4-nitro-2,5,6-trifluorbenzol (0,1 mol) werden in 400 ml eines Gemisches von Tetrahydrofuran und Essigsäureethylester (Volumenverhältnis 1:1) gelöst, und zu der Lösung werden 4,4 g Pd/C (Palladium/Kohlenstoff) gegeben. Dann wird die Lösung bei Raumtemperatur in einem Autoklaven zunächst - zur Abspaltung der Allylschutzgruppe - 6 h intensiv gerührt. Anschließend wird Wasserstoff eingeleitet und unter 1 bar Druck hydriert; nach 3 Tagen wird die Reaktion beendet. Die gelbe Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet.

Charakterisierung:
- Massenspektrum: Molekülpeak bei 340
- Elementaranalyse:

| | | | |
|---|---|---|---|
| Theoretischer Wert (in %) | C: 42,4 | H: 1,8 | N: 8,2 |
| Ermittelter Wert (in %) | C: 42,4 | H: 1,8 | N: 8,2 |

### Beispiel 12

### Herstellung von 1-(4-Nitro-2,3,5,6-tetrafluor-phenoxy)-3-benzyloxy-4-nitrobenzol

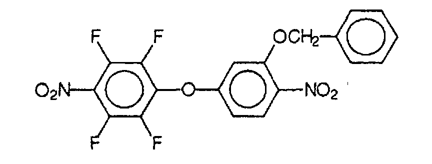

In einem Dreihalskolben mit Rührer werden 24,5 g 2-Benzyloxy-4-hydroxy-nitrophenol (0,1 mol) und 21,3 g Pentafluor-nitrobenzol (0,1 mol) in 300 ml γ-Butyrolacton gelöst. Dann gibt man portionsweise 30 g Kaliumcarbonat (0,22 mol) hinzu und rührt das Gemisch 24 h bei Raumtemperatur. Danach wird die Reaktionslösung über ein Faltenfilter abfiltriert und das Rohprodukt mit 200 ml Essigsäureethylester und 300 ml Wasser ausgeschüttelt. Die organische Phase wird dreimal mit Wasser gewaschen, über Natriumsulfat getrocknet und in einem Rotationsverdampfer auf die Hälfte eingeengt. Das ausgefallene Rohprodukt wird dann in einem Gemisch von Methylenchlorid und Petroleumbenzin (Volumenverhältnis 1:1) umkristallisiert und anschließend in einem Vakuumschrank 48 h unter Stickstoff bei 40°C/10 mbar getrocknet.

Charakterisierung:
- Massenspektrum: Molekülpeak bei 438
- Elementaranalyse:

| | | | |
|---|---|---|---|
| Theoretischer Wert (in %) | C: 52,1 | H: 2,3 | N: 6,4 |
| Ermittelter Wert (in %) | C: 52,1 | H:2,2 | N: 6,5 |

### Beispiel 13

### Herstellung von 1-(3-Hydroxy-4-nitro-2,5,6-trifluor-phenoxy)-3-benzyloxy-4-nitrobenzol

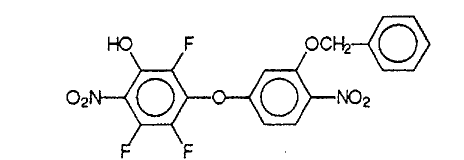

In einem Dreihalskolben mit Rückflußkühler und Rührer werden 43,8 g des entsprechend Beispiel 12 hergestellten 1-(4-Nitro-2,3,5,6-tetrafluor-phenoxy)-3-benzyloxy-4-nitrobenzol (0,1 mol) in einem Gemisch von 200 ml Dimethylsulfoxid und 30 ml Wasser gelöst. Zur Lösung werden portionsweise 15 g Kaliumcarbonat (0,11 mol) und 10 g Kaliumhydrogencarbonat (0,1 mol) gegeben, dann wird 24 h bei 80°C gerührt. Danach wird die Reaktionslösung über ein Faltenfilter abfiltriert und das Rohprodukt mit 200 ml Essigsäureethylester und 400 ml Wasser ausgeschüttelt. Die organische Phase wird dreimal mit Wasser gewaschen, über Natriumsulfat getrocknet und in einem Rotationsverdampfer auf die Hälfte eingeengt. Das ausgefallene Reaktionsprodukt wird dann in einem Gemisch von Methylenchlorid und Petroleumbenzin (Volumenverhältnis 1:1) umkristallisiert und anschließend in einem Vakuumschrank 48 h unter Stickstoff bei 40°C/10 mbar getrocknet.

Charakterisierung:
- Massenspektrum: Molekülpeak bei 436
- Elementaranalyse:

| | | | |
|---|---|---|---|
| Theoretischer Wert (in %) | C: 52,3 | H: 2,5 | N: 6,4 |
| Ermittelter Wert (in %) | C: 52,3 | H:2,4 | N: 6,4 |

### Beispiel 14

### Herstellung von 3-(3-Hydroxy-4-amino-2,5,6-trifluor-phenoxy)-6-aminophenol

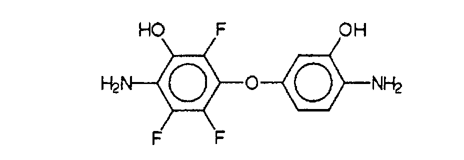

43,6 g des entsprechend Beispiel 13 hergestellten 1-(3-Hydroxy-4-nitro-2,5,6-trifluor-phenoxy)-3-benzyloxy-4-nitrobenzol (0,1 mol) werden in 400 ml eines Gemisches von Tetrahydrofuran und Essigsäureethylester (Volumenverhältnis 1:1) gelöst, und zu der Lösung werden 4,4 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die gelbe Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet.

Charakterisierung:
- Massenspektrum: Molekülpeak bei 286
- Elementaranalyse:

| | | | |
|---|---|---|---|
| Theoretischer Wert (in %) | C: 50,4 | H: 3,2 | N: 9,8 |
| Ermittelter Wert (in %) | C: 50,4 | H: 3,2 | N: 9,8 |

## Patentansprüche

1. o-Nitrophenolderivate und o-Nitrothiophenolderivate der Struktur 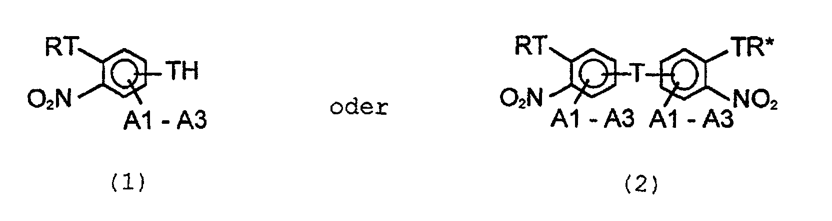 wobei folgendes gilt:
A¹ bis A³ sind - unabhängig voneinander - H, F, CH₃, CF₃, OCH₃, OCF₃, CH₂CH₃, CF₂CF₃, OCH₂CH₃ oder OCF₂CF₃;
T = O oder S;
R = Alkyl, Alkoxyalkyl, Alkenyl, Alkoxyalkenyl, Alkinyl oder Alkoxyalkinyl mit jeweils maximal sechs C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxy-alkenyl mit jeweils maximal vier aliphatischen C-Atomen;
R* = H oder R;
mit der Maßgabe, dass beim o-Nitro(thio)phenolderivat (1) R nicht Alkyl sein darf, wenn A¹ ein F-Atom in m-Stellung zur NO₂-Gruppe ist, A² und A³ die Bedeutung H haben und die TH-Gruppe in o-Stellung zur NO₂-Gruppe ist, und dass weiter, wenn R eine Allyl- oder Benzylgruppe ist, sich beim o-Nitrophenol-derivat (1) die TH-Gruppe in m-Stellung zur TR-Gruppe befindet; und dass weiter beim o-Nitrophenolderivat (1) R nicht Alkyl ist.

2. Verfahren zur Herstellung von o-Nitrophenolderivaten und o-Nitrothiophenolderivaten nach Anspruch 1, **dadurch gekennzeichnet, daß** - zur Herstellung einer Verbindung der Struktur (1) - bei einer Verbindung der Struktur 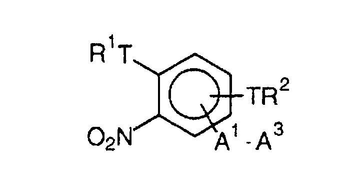 bei einer Temperatur von 20 bis 100°C selektiv der Rest R² abgespalten und durch H ersetzt wird, wobei folgendes gilt:
(a)
bei R¹ = Alkyl, Alkoxyalkyl, Alkenyl, Alkoxyalkenyl, Alkinyl oder Alkoxyalkinyl (mit jeweils maximal 6 C-Atomen)
und R² = Phenyl, Phenacyl oder Benzyl sowie Benzylalkyl, Benzylalkenyl, Benzyloxyalkyl, Benzyloxyalkenyl, Benzylalkoxyalkyl oder Benzylalkoxyalkenyl (mit jeweils maximal vier aliphatischen C-Atomen)
erfolgt die Spaltung mit Trifluoressigsäure, p-Toluolsulfonsäure, Lithium-, Natrium- oder Kalium-tert.-butoxid oder Lithium-, Natrium- oder Kaliumaluminiumhydrid oder durch Hydrierung mit Wasserstoff an Pd/C;
(b)
bei R¹ = Phenyl, Phenacyl oder Benzyl sowie Benzylalkyl, Benzylalkenyl, Benzyloxyalkyl, Benzyloxyalkenyl, Benzylalkoxyalkyl oder Benzylalkoxyalkenyl (mit jeweils maximal vier aliphatischen C-Atomen)
und R² = Alkyl, Alkoxyalkyl, Alkenyl, Alkoxyalkenyl, Alkinyl oder Alkoxyalkinyl (mit jeweils maximal 6 C-Atomen)
erfolgt die Spaltung mit einem Metall oder einer Mecallverbindung in einem protischen Lösemittel.

3. Verfahren zur Herstellung von o-Nitrophenolderivaten und o-Nitrothiophenolderivaten nach Anspruch 1, **dadurch gekennzeichnet, daß** eine Verbindung der Struktur 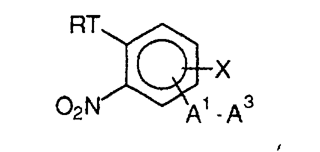 wobei R die angegebene Bedeutung hat und X Halogen ist, durch Umsetzung mit einem basischen Agens TH⁻ (mit T = O oder S) bei einer Temperatur von 20 bis 100°C
(a) bei einem Überschuß des basischen Agens in eine Verbindung der Struktur (1) oder
(b) bei einer stöchiometrischen Menge des basischen Agens in eine Verbindung der Struktur (2) mit R* = R
übergeführt wird.

4. Verfahren zur Herstellung von o-Nitrophenolderivaten und o-Nitrothiophenolderivaten nach Anspruch 1, **dadurch gekennzeichnet, daß** - zur Herstellung einer Verbindung der Struktur (2) mit R* = H - eine Verbindung der Struktur (1) mit einer Verbindung der Struktur 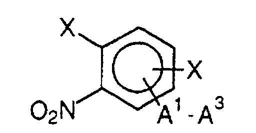 umgesetzt wird, wobei X Halogen ist,
und daß nachfolgend durch Umsetzung mit einem Überschuß eines basischen Agens TH⁻ (mit T = O oder S) bei einer Temperatur von 20 bis 100°C das Halogen X in o-Stellung zur NO₂-Gruppe durch TH ersetzt wird.

## Claims

1. o-Nitrophenol or o-nitrothiophenol derivatives of the structure 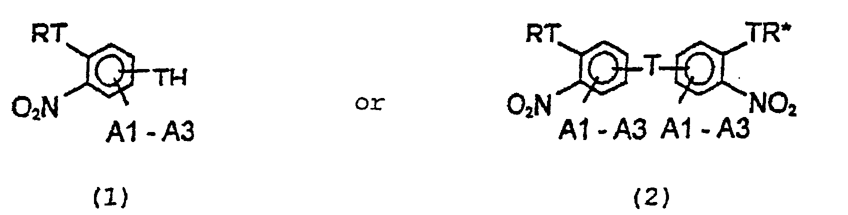 where:
A¹ to A³ are - independently of one another - H, F, CH₃, CF₃, OCH₃, OCF₃, CH₂CH₃, CF₂CF₃, OCH₂CH₃ or OCF₂CF₃;
T = O or S;
R = alkyl, alkoxyalkyl, alkenyl, alkoxyalkenyl, alkynyl or alkoxyalkynyl, each having a maximum of six carbon atoms, phenyl, phenacyl, benzyl and benzylalkyl, benzylalkenyl, benzyloxyalkyl, benzyloxyalkenyl, benzylalkoxyalkyl or benzylalkoxyalkenyl, each having a maximum of four aliphatic carbon atoms;
R* = H or R;
with the proviso that, in the o-nitro(thio)phenol derivative (1), R cannot be alkyl if A¹ is an F atom in the m-position to the NO₂ group, A² and A³ are H, and the TH group is in the o-position to the NO₂ group, and that furthermore, if R is an allyl or benzyl group, the TH group in the o-nitrophenol derivative (1) is in the m-position to the TR group; and that furthermore R in the o-nitrophenol derivative (1) is not alkyl.

2. Process for the preparation of o-nitrophenol and o-nitrothiophenol derivatives according to Claim 1, **characterized in that** - in order to prepare a compound of structure (1) - the radical R² is selectively removed from a compound of the structure 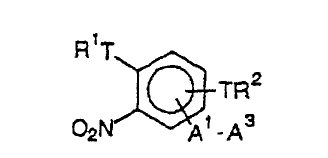 and is replaced by H at a temperature of from 20 to 100°C, where:
(a)
for R¹ = alkyl, alkoxyalkyl, alkenyl, alkoxyalkenyl, alkynyl or alkoxyalkynyl (each having a maximum of 6 carbon atoms)
and R² = phenyl, phenacyl or benzyl, or benzylalkyl, benzylalkenyl, benzyloxyalkyl, benzyloxyalkenyl, benzylalkoxyalkyl or benzylalkoxyalkenyl (each having a maximum of four aliphatic carbon atoms),
the removal of R² is carried out using trifluoroacetic acid, p-toluenesulphonic acid, lithium tert-butoxide, sodium tert-butoxide, potassium tert-butoxide, lithium aluminium hydride, sodium aluminium hydride or potassium aluminium hydride, or by hydrogenation using hydrogen on Pd/C;
(b)
for R¹ = phenyl, phenacyl or benzyl, or benzylalkyl, benzylalkenyl, benzyloxyalkyl, benzyloxy-alkenyl, benzylalkoxyalkyl or benzylalkoxyalkenyl (each having a maximum of four aliphatic carbon atoms),
and R² = alkyl, alkoxyalkyl, alkenyl, alkoxyalkenyl, alkynyl or alkoxyalkynyl (each having a maximum of 6 carbon atoms),
the removal of R² is carried out using a metal or a metal compound in a protic solvent.

3. Process for the preparation of o-nitrophenol and o-nitrothiophenol derivatives according to Claim 1, **characterized in that** a compound of the structure 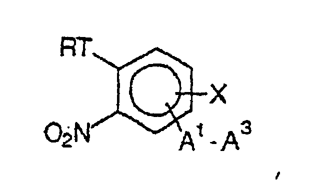 where R is as defined above and X is halogen, by reacting with a basic agent TH⁻ (where T = O or S) at a temperature of from 20 to 100°C, is converted
(a) in the presence of an excess of the basic agent, into a compound of structure (1) or
(b) in the presence of a stoichiometric amount of the basic agent, into a compound of structure (2) where R* = R.

4. Process for the preparation of o-nitrophenol and o-nitrothiophenol derivatives according to Claim 1, **characterized in that** - in order to prepare a compound of structure (2) where R* = H - a compound of structure (1) is reacted with a compound of the structure 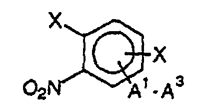 where X is halogen,
and then the product is reacted with an excess of a basic agent TH⁻ (where T = O or S) at a temperature of from 20 to 100°C in order to replace the halogen X in the o-position to the NO₂ group by TH.

## Revendications

1. Composés o-nitrophénolique et o-nitrothiophénolique de structure 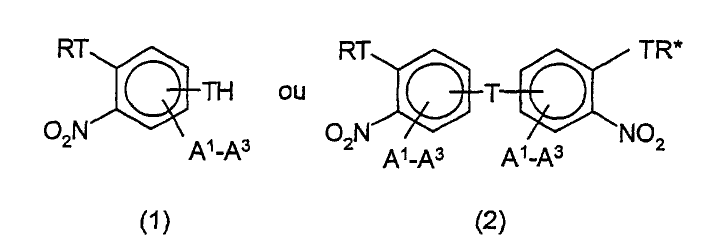 dans lesquelles :
A¹ à A³ sont indépendamment les uns des autres - H, F, CH₃, CF₃, OCH₃, OCF₃, CH₂CH₃, CF₂CF₃, OCH₂CH₃ ou OCF₂CF₃ ;
T= O ou S ;
R = alcoyle, alcoxyalcoyle, alcényle, alcoxyalcényle, alcynyle ou alcoxyalcynyle ayant respectivement au maximum 6 atomes de carbone, phényle, phénacyle, benzyle et benzylalcoyle, benzylalcényle, benzyloxyalcoyle, benzyloxyalcényle, benzylalcoxyalcoyle ou benzylalcoxyalcényle ayant respectivement au maximum 4 atomes de carbone aliphatiques ;
R* = H ou R ;
pourvu que, pour le composé o-nitro(thio)phénolique (1), R ne soit pas alcoyle lorsque A¹ est un atome de F en position métha par rapport au groupe NO₂, A² et A³ ont la signification H et le groupe TH est en la position ortho par rapport au groupe NO₂ et pourvu que, en outre, lorsque R est un groupe allyle ou benzyle, le groupe TH dans le composé (1) o-nitrophénolique se trouve en la position métha par rapport au groupe TR ; et pourvu que, en outre, dans le composé (1) o-nitrophénolique, R ne soit pas alcoyle.

2. Procédé de préparation de composés o-nitrophénoliques et de composés o-nitrothiophénoliques suivant la revendication 1, **caractérisé en ce que**, pour préparer un composé de structure (1), on élimine dans un composé de structure 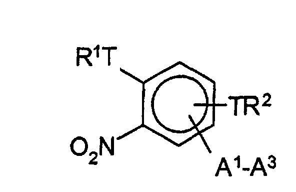 à une température de 20 à 100°C sélectivement le radical R² et on le remplace par H dans les conditions suivantes :
(a)
lorsque R¹ = alcoyle, alcoxyalcoyle, alcényle, alcoxyalcényle, alcynyle ou alcoxyalcynyle (ayant respectivement 6 atomes de carbone au maximum)
et lorsque R² = phényle, phénacyle ou benzyle ainsi que benzylalcoyle, benzylalcényle, benzyloxyalcoyle, benzyloxyalcényle, benzylalcoxyalcoyle ou benzylalcoxyalcényle (ayant respectivement 4 atomes de carbone aliphatiques au maximum),
l'élimination s'effectue par l'acide trifluoroacétique, l'acide p⁻toluènesulfonique, le butylate tertiaire de lithium, le butylate tertiaire de sodium ou le butylate tertiaire de potassium ou l'hydrure de lithium et d'aluminium, l'hydrure de sodium et d'aluminium ou l'hydrure de potassium et d'aluminium ou par hydrogénation par l'hydrogène sur Pd/C ;
(b)
lorsque R¹ = phényle, phénacyle ou benzyle ainsi que benzylalcoyle, benzylalcényle, benzyloxyalcoyle, benzyloxyalcényle, benzylalcoxyalcoyle ou benzylalcoxyalcényle (ayant respectivement 4 atomes de carbone aliphatiques au maximum),
et lorsque R² = alcoyle, alcoxyalcoyle, alcényle, alcoxyalcényle, alcynyle ou alcoxyalcynyle (ayant respectivement 6 atomes de carbone au maximum)
l'élimination s'effectue par un métal ou par un composé métallique dans un solvant protique.

3. Procédé de préparation de composés o-nitrophénoliques et de composés o-nitrothiophénoliques **caractérisé en ce que** l'on transforme un composé de structure, 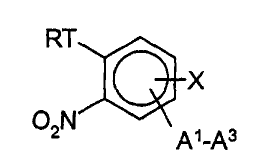 dans laquelle R a la signification indiquée et X est un halogène, par réaction sur un agent TH⁻ basique (avec T = O ou S) à une température de 20 à 100°C
(a) pour un excès de l'agent basique en un composé de structure (1) ou
(b) pour une quantité stoechiométrique de l'agent basique en un composé de structure (2) avec R* = R.

4. Procédé de préparation de composés o-nitrophénoliques et de composés o-nitrothiophénoliques suivant la revendication 1, **caractérisé en ce que** pour préparer un composé de structure (2) avec R* = H, on fait réagir un composé de structure (1) sur un composé de structure 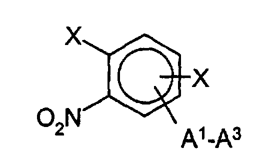 dans laquelle X est un halogène et **en ce qu'**ensuite, on remplace par réaction sur un excès d'un agent TH⁻ basique (avec T = O ou S) à une température de 20 à 100°C, l'halogène X en position ortho par rapport au groupe NO₂ par TH.
